Europäisches Patentamt

European Patent Office

Office européen des brevets

(19)

(11) Publication number: **0 032 299**
**B1**

# EUROPEAN PATENT SPECIFICATION

(12)

(45) Date of publication of patent specification: **01.02.84**

(51) Int. Cl.³: **C 07 C 120/02**

(21) Application number: **80304527.7**

(22) Date of filing: **15.12.80**

(54) Production of dicyanobutene.

(30) Priority: **11.01.80 GB 8001008**

(43) Date of publication of application:
**22.07.81 Bulletin 81/29**

(45) Publication of the grant of the patent:
**01.02.84 Bulletin 84/5**

(84) Designated Contracting States:
**BE DE FR GB IT NL**

(56) References cited:
**DE - A - 2 642 449**

(73) Proprietor: **IMPERIAL CHEMICAL INDUSTRIES PLC**
**Imperial Chemical House Millbank**
**London SW1P 3JF (GB)**

(72) Inventor: **Waddan, Dhafir Yusuf**
**35 Linwood Avenue**
**Stokesley Middlesbrough Cleveland (GB)**

(74) Representative: **Taylor, Michael et al,**
**Imperial Chemical Industries PLC Legal**
**Department: Patents Po Box 6**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Production of dicyanobutene

The present invention relates to the production of dicyanobutene.

Dicyanobutene is industrially important because it is convertible to adiponitrile which is an intermediate in the manufacture of nylon. In our British Patent No 1,541,443 we have described a process for the production of dicyanobutene by the reaction of butadiene with hydrogen cyanide and oxygen, or a molecular oxygen-containing gas, in the presence of a catalyst comprising copper ions and halide ions and of a solvent for the catalyst. The process is described as being carried out by passing the butadiene and hydrogen cyanide in vapour form together with the oxygen, or oxygen-containing gas, through a liquid comprising the catalyst and solvent or alternatively, the butadiene and hydrogen cyanide may be kept in the liquid phase under pressure with the catalyst and solvent and the oxygen, or oxygen-containing gas, passed through. Although in both these methods of operation the budadiene, hydrogen cyanide and oxygen are in contact with the catalyst at one and the same time the possibility is also envisaged in 1,541,443 of a two stage method of operation in which butadiene and hydrogen cyanide on the one hand and oxygen on the other hand are alternately passed through the liquid comprising the catalyst and solvent.

One disadvantage of a method of operation in which the hydrogen cyanide, butadiene and oxygen are all present together is the combined explosion (butadiene plus oxygen) and poison (hydrogen cyanide) hazard. This may be overcome to some extent by the two stage method of operation but we have now found that even more benefits accrue if the process is operated in three sequential stages. By operating in this manner not only are the hazards of the process reduced but, unexpectedly, less byproduct is formed.

Accordingly, the invention comprises a process for the manufacture of dicyanobutene which comprises reacting butadiene with hydrogen cyanide in the presence of a catalyst which is activated by contact with molecular oxygen or a molecular oxygen-containing gas and which comprises copper ions and halide ions, the reaction being carried out in the presence of a solvent for the catalyst, in which the hydrogen cyanide, oxygen and butadiene are each separately contacted with the catalyst in the sequence

(a) oxygen, butadiene, hydrogen cyanide, or

(b) oxygen, hydrogen cyanide, butadiene,

The copper ions in the catalyst used in the process of the invention may be added in the cuprous or cupric form. We believe that the active catalyst is a complex of cupric, halide and hydrogen cyanide which is reduced back to cuprous by the butadiene/hydrogen cyanide reaction. The oxygen oxidises the cuprous back again to the cupric form, this reaction taking place before or after the copper has complexed with the hydrogen cyanide. The copper may be added to the reaction mixture as a halide, for example as cuprous or cupric chloride, bromide or iodide (or as any mixture thereof) since this will ensure the presence of halide in addition to copper, but this is not essential. Other copper salts may be used, especially the salts of organic acids, more especially the salts of aliphatic carboxylic acids and particularly the salts of alkane carboxylic acids having from 2 to 6 carbon atoms. As examples of such copper salts there may be mentioned copper formate, acetate, propionate, butyrate, lactate, glycollate, acetylacetonate, naphthenate, stearate and benzoate. Moreover, other sources of halide ion may be used for example alkali metal and ammonium chloride, bromide and iodide as well as hydrogen chloride, bromide and iodide and chlorine, bromine and iodine themselves. Further, organic chloride, bromine and iodine compounds may be used as the halide source, for example tetrabromoethane, chloracetic acid, bromoacetic acid, acetylbromide, dichlorobutene and dibromobutene, as well as hydrochlorides, hydrobromides and hydroiodides or organic bases and quaternary ammonium bromides and iodides. It is advantageous also for there to be present an alkali metal salt, for example a sodium, potassium or especially a lithium salt, or an alkaline earth metal salt, for example a beryllium, magnesium, calcium or barium salt. Such a salt is preferably a chloride, bromide or iodide, but may be, for example, an organic acid salt, especially a salt with one of the organic acids specified above as forming suitable copper salts. As examples of such salts there may be mentioned, lithium chloride, lithium bromide, lithium iodide, lithium acetate, lithium propionate, sodium bromide, sodium iodide, sodium acetate, potassium bromide, potassium iodide, potassium acetate and magnesium bromide.

Preferably the halide ion in the catalyst consists of a mixture of chloride and/or bromide ion with iodide ion, since this gives a more active catalyst. Mixtures of bromide with iodide ion are particularly suitable. The uptake of oxygen may be assisted by the presence of oxygen carriers, for example, manganese compounds, eg manganese gluconate.

As solvents for the catalyst there may be used a wide variety of compounds. The basic requirements are that the catalyst components shall dissolve to a greater or lesser extent in the solvent and that the solvent shall not interfere with the reaction and shall not itself be extensively changed by the reaction. Thus olefinically unsaturated compounds which react with hydrogen cyanide under the reaction conditions are unsuitable, as are solvents, for example mercaptans, which would be oxidised by the oxygen-containing gas under the reaction conditions. The solvent should preferably be liquid at the reaction temperature and pressure. However, compounds which are normally solid under the reaction conditions may be used dissolved in another solvent.

Water is a suitable solvent as are many organic compounds. Particularly suitable classes of organic compounds include nitriles, alcohols, phenols, ethers, acids, ketones and amides. Suitable nitriles include aliphatic, cycloaliphatic, araliphatic and aromatic nitriles. More especially they include alkyl nitriles and alkylene dinitriles, particularly those having from 1 to 6 carbon atoms in the alkyl or alkylene residue, for example acetonitrile, propionitrile, butyronitrile, hexanonitrile, glutarodinitrile, adiponitrile, dicyanobutene and succinidinitrile, alkenyl nitriles, for example acrylonitrile, methacrylonitrile, butenenitriles, methyl butenenitriles and pentenenitriles, higher polynitriles, for example tetracyanoethylene, cycloalkyl nitriles, for example cyclohexyl cyanide, aralkyl nitriles, for example benzyl cyanide and $\alpha$, $\alpha'$-xylylene dinitrile and aryl nitriles, for example benzonitriles, tolunitriles, phthalodinitrile and terephthalodinitrile. Particularly suitable nitriles include acetonitrile, propionitrile and adiponitrile.

Suitable alcohols include aliphatic, cycloaliphatic and araliphatic alcohols. More especially they include alkanols, particularly those having from 1 to 6 carbon atoms, for example methanol, ethanol, n-propanol, isopropanol, butanols, pentanols and hexanols, alkandiols, particularly those having from 1 to 6 carbon atoms, for example ethylene glycol, propane-1,2-diol, propane-1,3-diol, butane-1,4-diol, pentane-diols and hexanediols, alkanepolyols, for example glycerol and trimethylolpropane, aralkanols, for example benzyl alcohol and 2-phenylethanol, and cycloalkanols, for example cyclopentanol, methyl-cyclopentanols, cyclohexanol and methylcyclohexanols. Particularly suitable alcohols include ethanol and isopropanol.

Suitable phenols include phenol itself, alkylphenols, for example cresols, ethylphenols and xylenols and halogenophenols, especially chlorophenols and di- and tri-chloro-henols. m-Cresol is a particularly suitable phenol.

Suitable ethers include aliphatic ethers araliphatic ethers, aromatic ethers and cyclic ethers. More especially they include dialkyl ethers, for example di-isopropyl ether and methyl butyl ether, bis-ethers and polyethers for example 1,2-dimethoxyethane, 1,2-dimethyoxypropane and diethyleneglycol dimethylether (diglyme), cyclic ethers, for example tetrahydrofuran, tetrahydropyran, dioxan, diphenylene oxide and crown ether (6, 7, 9, 10, 17, 18, 20, 21-octahydrodibenzo (b, k) (1, 4, 7, 10, 13, 16)-hexaoxycyclooctadecene), alkyl aryl ethers, for example anisole and phenetole, diaralkyl ethers, for example dibenzyl ether, and diaryl ethers for example diphenyl oxide. Dimethyoxyethane, diglyme and tetrahydrofuran are particularly suitable ethers.

Suitable organic acids are especially the carboxylic acids. Suitable carboxylic acids include aliphatic, cycloaliphatic, araliphatic and aromatic carboxylic acids. More especially they include alkane carboxylic acids, particularly those having from 2 to 6 carbon atoms in the alkane residue, for example acetic acid, propionic acid, butyric acid, isobutyric acid, valeric acid or caproic acid, cycloalkane carboxylic acids, for example cyclohexane carboxylic acid and cyclohexylacetic acid, aralkyl carboxylic acids, for example phenylacetic acid, aryl carboxylic acids, for example benzoic acid, toluic acids and anisic acids, and naphthenic acids. Acetic acid is particularly suitable.

Suitable ketones include aliphatic, cycloaliphatic, araliphatic, aromatic and cyclic ketones. More especially they include dialkyl ketones, particularly those having from 1 to 6 carbon atoms in the alkyl residues, for example acetone, methyl ethyl ketone and methyl isobutyl ketone, diketones, for example acetylacetone, cyclic ketones, for example cyclopentanone, methylcyclopentanone, cyclohexanone and methylcyclohexanone, alkyl aryl ketones, for example acetophenone, and diaryl ketones, for example benzophenone. Acetone and acetylacetone are particularly suitable ketones.

Suitable amides include in particular aliphatic carboxylic amides and their N-substituted derivatives. More especially they include carboxylic amides, particularly those having from 1 to 4 carbon atoms, and their N-alkyl and N,N-dialkyl derivatives especially those having from 1 to 4 carbon atoms in the alkyl residues, for example formamide, N-methylformamide, N,N-dimethylformamide, acetamide, N,N-dimethylacetamide and propionamide. They also include cyclic amides for example N-methyl-2-pyrrolidone. Dimethylformamide is a particularly suitable amide.

Suitable solvents also include compounds which contain two or more of the functional groups which characterise, respectively, the said nitriles, alcohols, phenols, ethers, acids, ketones and amides, or contain one or more of the said functional groups in combination with some other group. Such compounds include, for example, ether-alcohols, for example ethylene glycol monomethyl and monoethyl ether, nitrile-acids, for example cyanoacetic acid and $\alpha$-cyanovaleric acid, halogeno-acids, for example chloroacetic acids, dichloroacetic acid and trichloroacetic acid and nitrileesters, for example ethyl cyanoacetate.

Other suitable solvents include esters, especially the esters formed from the alcohols and acids already described as suitable solvents. Particularly suitable esters are the lower alkylesters (eg where lower alkyl has from 1 to 4 carbon atoms) of aliphatic mono- or di-carboxylic acids especially those having from 1 to 6 carbon atoms, for example methyl acetate, ethyl acetate, iso-propyl acetate, ethyl propionate, methyl butyrate, dimethyl succinate, dimethyl glutarate and diethyl adipate.

Other suitable solvents include hydrocarbons and halogenated hydrocarbons. Such solvents include both aliphatic, cycloaliphatic and aromatic hydrocarbons, and their halogenated derivatives, for example hexane, cyclohexane, benzene, toluene, xylene, chloroform, carbon tetrachloride, trichloroethylene, tetrachlorethane, dibromoethane, chlorobenzene, bromobenzene, dichlorobenzene trichlorobenzene and diphenyl.

Other suitable solvents include thioethers, that is sulphides, including cyclic sulphides, for example dimethyl sulphide, diethyl sulphide, dipropyl sulphide, dibutyl sulphide, diamyl sulphide, dihexyl sulphide, methyl ethyl sulphide, thiophen, tetrahydrothiophen, pentamethylene sulphide, dicyclohexyl sulphide, dibenzyl sulphide, diphenyl sulphide, ditolyl sulphide and thiodiglycol.

Other suitable solvents include sulphoxides and sulphones, especially dialkyl sulphoxides and sulphones, particularly where the alkyl group has from 1 to 6 carbon atoms, and cyclic sulphoxides and sulphones, for example dimethyl sulphoxide, diethyl sulphoxide, diethyl sulphone, dimethyl sulphone, tetramethylene sulphoxide, tetramethylene sulphone (sulpholane) and pentamethylene sulphoxide and pentamethylene sulphone.

The solvents may be used singly or in admixture with each other in any convenient proportions. Moreover the solvents may be used in admixture with organic compounds which are not in themselves solvents for the catalyst.

The oxygen may be used as such or in admixture with nonreactive gases such as nitrogen. Air is a particularly suitable oxygen-containing gas, but mixtures of oxygen and nitrogen with a higher or lower proportion of oxygen than that of the air may also be used.

The reaction stages are conveniently carried out at temperatures within the range 10° to 150°C. The reaction involving oxygen may be carried out at a higher temperature within this range eg at ambient temperature to 110°C while the reactions involving butadiene and hydrogen cyanide may be operated at a lower temperature eg at ambient temperature to 60°C. For operating convenience however it may be preferred for all three stages to be carried out at the same temperature. The reaction stages may be carried out at atmospheric pressure or at pressures above or below that of the atmosphere. The stage in which oxygen is a reactant may advantageously be operated under pressure, and pressures may, for example, be up to about 50 bar. Pressures in the range 2 to 10 bar absolute are very suitable, and again, for operating convenience all three stages may be operated at the same pressure.

The butadiene used in the process of our invention may contain other constituents. For example the butadiene may be admixed with other $C_4$ hydrocarbons, for example butenes and butane. Instead of using butadiene itself, a crude $C_4$ stream from a cracker containing possibly less than 50% of butadiene may be used as the feed in our process to produce dicyanobutene.

Water is formed in the process of our invention, and it may be desirable, for example when using organic solvents, to remove the water from the reaction system. The water is usually taken up into the reactant gas stream and is preferably condensed out from the effluent gas stream at least in part, prior to any recycle.

The catalyst is used in catalytic amount. The amount of copper ion may vary, for example, from 0.001 mole to 0.2 mole per mole of butadiene, although higher proportions are not excluded. The amount of halide ion in total may vary, for example, within the same molar range, although we prefer that there is at least one mole of halide ion per mole of copper ion. Where, as we prefer, the halide ions consist of a mixture of chloride and/or bromide ion with iodide ion the relative proportions of iodide to chloride and/or bromide may vary within wide limits, for example the iodide may vary from 0.1% to 90% of the combined chloride, bromide andiodide on a molar basis, but we prefer the iodide proportion to be between 1% and 10%. The amount of solvent used may vary widely. There should preferably be at least one mole of solvent per mole of copper ion, and amounts between 5 moles and 100 moles are convenient. When an alkali metal or alkaline earth metal compound is present it may be used in amounts up to several times the molar amount of copper, for example in amounts of from 0.5 to 15 molers per mole of copper.

The dicyanobutene obtained as the product of our process is normally present in the liquid reaction mixture and may be separated therefrom by conventional methods, for example by fractional distillation under reduced pressure, by extraction with solvents, or by a combination of such methods.

The dicyanobutene product of our process is principally 1,4-dicyanobutene. This is a valuable intermediate, since it may, by hydrogenation of the double bond, be converted to adiponitrile which itself, on hydrogenation of the nitrile groups, give hexamethylene diamine, an intermediate useful in the manufacture of polymers, for example polyurethanes and especially polyamides, in particular polyamides made by polycondensation with dicarboxylic acids, for example with adipic acid to give polyhexamethylene adipamide (nylon 6.6) useful for the manufacture of mouldings and for melt-spinning into synthetic fibres.

The invention is illustrated but not limited by the following Examples.

Example 1

The apparatus consisted of a 250 ml stainless steel autoclave fitted with a glass liner and a magnetic stirrer. The catalyst comprised 2 grams of copper bromide and 0.2 grams of iodine dissolved in 20 mls of propionitrile and this mixture was placed in the glass liner in the autoclave.

The reaction in the autoclave was carried out cyclically each cycle consisting of three stages viz (1) The autoclave was pressurised to 100 psi with a 50/50% by volume mixture of nitrogen and

4

O 032 299

oxygen. The autoclave was then held at room temperature for 1 hour and the pressure released.

(2) Butadiene (3 mls) was then added to the autoclave and the autoclave contents heated to 50°C and held at this temperature for one hour. The contents were then cooled to room temperature and excess butadiene vented off.

(3) Hydrogen cyanide (3 mls) was next added and the autoclave and the contents again heated to 50°C and held at this temperature for 1 hour. Finally, the contents were again cooled to room temperature and excess hydrogen cyanide vented off.

After 60 cycles with no apparent loss in catalyst activity the experiment was stopped. 25.8 grams of 1,4-dicyanobutene-2 were recovered equivalent to 17.5 moles/mole copper bromide. Gas-liquid chromatographic analysis of the product showed that it contained 0.04 moles cyanogen/mole of dicyanobutene. The conversion of butadiene to dicyanobutene was 95%.

Example 2

Example 1 was repeated using the following reaction sequence:-

(1) Hydrogen cyanide (3 mls) reacted for 1 hour at 50°C.
(2) Oxygen/nitrogen (50/50) reacted at 100 psi for 1 hour at room temperature.
(3) Butadiene (3 mls) reacted for 1 hour at 50°C.

A similar number of cycles to Example 1 was achieved to give a similar amount of product of comparable purity.

**Claims**

1. A process for the manufacture of dicyanobutene which comprises reacting butadiene with hydrogen cyanide in the presence of a catalyst which is activated by contact with molecular oxygen or a molecular oxygen-containing gas and which comprises copper ions and halide ions, the reaction being carried out in the presence of a solvent for the catalyst characterised in that the hydrogen cyanide, oxygen and butadiene are each separately contacted with the catalyst in the sequence

    (a) oxygen, butadiene, hydrogen cyanide, or
    (b) oxygen, hydrogen cyanide, butadiene.

2. A process according to Claim 1 characterised in that the copper is added to the reaction mixture as a cuprous or cupric halide or the copper salt of an organic acid.

3. A process according to Claim 1 or Claim 2 characterised in that the halide ions are provided as alkali metal or ammonium halide, hydrogen halide, a halogen or an organic halogen compound.

4. A process according to any one of the preceding claims characterised in that the halide ion is a mixture of chloride and/or bromide ion with iodide ion.

5. A process according to any one of the preceding claims characterised in that the solvent is a nitrile, an alcohol, a phenol, an ether, an acid, an ester, a ketone, an amide, a thioether, a sulphoxide or sulphone, or a hydrocarbon or halogenated hydrocarbon or water.

6. A process according to Claim 5 characterised in that the solvent is acetonitrile, propionitrile, adiponitrile, glutaronitrile or succinidinitrile.

7. A process according to any one of the preceding claims characterised in that the temperature is in the range 10° to 150°C.

8. A process according to Claim 7 characterised in that the reaction stage involving oxygen is carried out at ambient temperature to 110°C and the stages involving butadiene and hydrogen cyanide at ambient temperature to 60°C.

9. A process according to any one of the preceding claims characterised in that the pressure is up to 50 bar.

10. A process according to any one of the preceding claims characterised in that the amount of copper present is 0.001 mole to 0.2 mole per mole of butadiene and the amount of halide ion is at least one mole per mole copper ion.

**Patentansprüche**

1. Verfahren zur Herstellung von Dicyanbuten, bei dem Butadien in Gegenwart eines Katalysators, der durch Kontakt mit molekularem Sauerstoff oder einem molekularen Sauerstoff enthaltenden Gas aktiviert ist und Kupferionen und Halogenidionen enthält, mit Blausäure umgesetzt wird, wobei die Umsetzung in Gegenwart eines Lösungsmittels für den Katalysator durchgeführt wird, dadurch gekennzeichnet, daß die Blausäure, der Sauerstoff und das Butadien jeweils getrennt in der Reihenfolge

    (a) Sauerstoff, Butadien, Blausäure oder
    (b) Sauerstoff, Blausäure, Butadien

mit dem Katalysator in Kontakt gebracht werden.

2. Verfahren nach Anspruch 1, dadurch gekennzeichnet, daß des Kupfer in Form eines Kupfer(I)- oder Kupfer(II)-halogenids oder des Kupfersalzes einer organischen Säure zu der Reaktionsmischung zugegeben wird.

3. Verfahren nach Anspruch 1 oder 2, dadurch gekennzeichnet, daß die Halogenidionen in Form von Alkalimetall- oder Ammoniumhalogenid, Halogenwasserstoff, eines Halogens oder einer organischen Halogenverbindung eingesetzt werden.

4. Verfahren nach einem der vorhergehenden Ansprüche, dadurch gekennzeichnet, daß die Halogenidionen eine Mischung von Chlorid- und/oder Bromidionen mit Jodidionen sind.

5. Verfahren nach einem der vorhergehenden Ansprüche, dadurch gekennzeichnet, daß das Lösungsmittel ein Nitril, ein Alkohol, ein Phenol, ein Ether, eine Säure, ein Ester, ein Keton, ein Amid, ein Thioether, ein Sulfoxid oder Sulfon oder ein Kohlenwasserstoff oder halogenierter Kohlenwasserstoff oder Wasser ist.

6. Verfahren nach Anspruch 5, dadurch gekennzeichnet, daß das Lösungsmittel Acetonitril, Propionitril, Adiponitril, Glutarsäuredinitril oder Bernsteinsäuredinitril ist.

7. Verfahren nach einem der vorhergehenden Ansprüche, dadurch gekennzeichnet, daß die Temperatur in dem Bereich von 10° bis 150°C liegt.

8. Verfahren nach Anspruch 7, dadurch gekennzeichnet, daß die Reaktionsstufe, in die Sauerstoff einbezogen ist, bei einer Temperatur von Umgebungstemperatur bis 110°C durchgeführt wird und daß die Stufen, in die Butadien und Blausäure einbezogen sind, bei einer Temperatur von Umgebungstemperatur bis 60°C durchgeführt werden.

9. Verfahren nach einem der vorhergehenden Ansprüche, dadurch gekennzeichnet, daß der Druck bis zu 50 bar beträgt.

10. Verfahren nach einem der vorhergehenden Ansprüche, dadurch gekennzeichnet, daß die Menge des vorhandenen Kupfers 0,001 mol bis 0,2 mol pro Mol Butadien und die Menge der Halogenidionen mindestens ein Mol pro Mol Kupferionen beträgt.

## Revendications

1. Procédé de production de dicyanobutène qui comprend la réaction du butadiène avec le cyanure d'hydrogène en présence d'un catalyseur qui est activé par contact avec de l'oxygène moléculaire ou un gaz contenant de l'oxygène moléculaire et qui comprend des ions cuivre et des ions halogénure, la réaction étant exécutée en présence d'un solvant pour le catalyseur, caractérisé en ce qu'on met le cyanure d'hydrogène, l'oxygène et le butadiène chacun en contact séparément avec le catalyseur dans la séquence
(a) oxygène, butadiène, cyanure d'hydrogène ou
(b) oxygène, cyanure d'hydrogène, butadiène.

2. Procédé suivant la revendication 1, caractérisé en ce que le cuivre est ajouté au mélange de réaction sous la forme d'un halogénure cuivreux ou cuivrique ou du sel de cuivre d'un acide organique.

3. Procédé suivant la revendication 1 ou 2, caractérisé en ce que les ions halogénure sont apportés sous forme d'un halogénure de métal alcalin ou d'ammonium, d'un halogénure d'hydrogène, d'un halogène ou d'un composé organique halogéné.

4. Procédé suivant l'une quelconque des revendications précédentes, caractérisé en ce que l'ion halogénure est un mélange d'ion chlorure et/ou bromure avec l'ion iodure.

5. Procédé suivant l'une quelconque des revendications précédentes, caractérisé en ce que le solvant est un nitrile, un alcool, un phénol, un éther, un acide, un ester, une cétone, un amide, un thioéther, un sulfoxyde ou une sulfone, un hydrocarbure ou hydrocarbure halogéne ou de l'eau.

6. Procédé suivant la revendication 5, caractérisé en ce que le solvant est l'acétonitrile, le propionitrile, l'adiponitrile, le glutaronitrile ou le succinodinitrile.

7. Procédé suivant l'une quelconque des revendications précédentes, caractérisé en ce que la température est située dans l'intervalle de 10 à 150°C.

8. Procédé suivant la revendication 7, caractérisé en ce que le stade de réaction mettant l'oxygène en jeu est exécuté de la température ambiante à 110°C et les stades mettant le butadiène et le cyanure d'hydrogène en jeu, de la température ambiante à 60°C.

9. Procédé suivant l'une quelconque des revendications précédentes, caractérisé en ce que la pression s'élève jusqu'à 50 bars.

10. Procédé suivant l'une quelconque des revendications précédentes, caractérisé en ce que la quantité de cuivre en présence est de 0,001 à 0,2 mole par mole de butadiène et la quantité d'ion halogénure est d'au moins 1 mole par mole d'ion cuivre.